# EUROPEAN PATENT APPLICATION

(11) **EP 1 415 658 A1**
(43) Date of publication of application: **06.05.2004**
(21) Application number: 02746077.3
(22) Date of filing: 16.07.2002
(51) Int. Cl.: A61K 35/84, A61P 25/28, A61P 25/00, A61P 43/00, A23L 1/30, A23L 2/38, A23K 1/18

(54) **REMEDIES**

(30) Priority: 16.07.2001 JP 2001215524
(71) Applicant: TAKARA BIO INC., Otsu-shi, Shiga 520-2193 (JP)
(72) Inventor: OHNOGI, Hiromu, Kusatsu-shi, Shiga 525-0025 (JP); SUGIYAMA, Katsumi, Otsu-shi, Shiga 520-2134 (JP); SAGAWA, Hiroaki, Kusatsu-shi, Shiga 525-0025 (JP); KATO, Ikunoshin, Shigaraki-cho,koka-gun, Shiga 529-1851 (JP)
(74) Representative: VOSSIUS & PARTNER
(86) International application number: PCT/JP2002/007193
(87) International publication number: WO 2003/007977

(57) **Abstract**

According to the present invention, there is provided a therapeutic agent or prophylactic agent that is effective for a disease requiring enhancement of nerve growth factor production for treatment or prevention, comprising as an effective ingredient an aqueous extract of mushrooms. These agents are usable in the treatment or prevention of a disease requiring enhancement of nerve growth factor production such as dementia or nerve disorder.

In addition, according to the present invention, there is provided an enhancing agent for growth factor production, or a food, beverage or feed for enhancing nerve growth factor production, each comprising the above-mentioned effective ingredient. According to the food or beverage, by taking as a foodstuff on a daily basis, the symptoms of a disease requiring enhancement of nerve growth factor production can be ameliorated or the like. In addition, the feed is useful for maintaining homeostasis of a living body by its enhancing action for nerve growth factor production.

## Description

### TECHNICAL FIELD

The present invention relates to a medicament, food, beverage or feed for enhancing nerve growth factor production, comprising as an effective ingredient an aqueous extract of a mushroom.

### BACKGROUND ART

Recently, various reports have been made on physiological activities owned by the mushrooms. Especially, as to the enhancing action for nerve growth factor (hereinafter simply referred to as "NGF" in some cases) production, the existence of enhancing substances for NGF production in an extract of fruit bodies of *Hericium erinaceum*, *Mycoleptodonoides aitchisonii*, *Dictyophora indusiata* and *Sarcodon scabrosus* has been reported (Japanese Patent Laid-Open Nos. Hei 9-308458, Hei 9-124541, Hei 9-20714 and Hei 11-269125). However, in these enhancing substances for NGF production or the extraction procedure for the extract having enhancing activity for NGF production, only a nonpolar solvent has been used as an extraction solvent, and moreover its activity is not that can be satisfied. In addition, there has not been reported so far on the enhancing activity for NGF production of an aqueous extract of a mushroom.

Nerve cells play a principal role for sustaining psychoactivities of human being such as intellectual functions, memory, emotions and behaviors. It has been thought that the differentiation, survival and exhibition of functions of the nerve cells which are the foundations of these psychoactivities need a neurotrophic factor specific for each nerve cell. Among the neurotrophic factors, one of which existence and function have been firstly elucidated is NGF, and currently, there have been found a brain-derived-neurotrophic factor, neurotrophin-3, neurotrophin-4/5, and the like.

NGF is a neurotrophic factor of a large cellular cholinergic nerve cell of basal portion of the forebrain, so that its association with Alzheimer's dementia has been remarked [*Pharmacia*, Vol. 22, No. 2, 147-151 (1986), *Ronen Seishin Igaku (Senile Psychiatry),* Vol. 3, No. 6, 751-758 (1986)].

Alzheimer's dementia refers to a disease that gives a pathological finding such as senile plaque or Alzheimer's fibrillar changes, which are accompanied by a clinical picture such as developmental disability, manic state, tonic seizures of lower limbs, or epileptic seizure, and is one disease of senile dementia. The Alzheimer's dementia tends to be increasing in recent aging society, so that a larger societal interest has been drawn thereto. However, there has not yet been found a satisfactory method for ameliorating or treating such symptoms. There has not yet been also found such a method for juvenile Alzheimer's diseases.

In the brain of a patient with Alzheimer's dementia, there has been found a dramatic denaturation, a drastic lowering of the activity of choline acetyl transferase (CAT), in the basal portion of the forebrain centering about Meynert's basal nuclei [*Annu*. *Rev. Neurosci*., Vol. 3, 77 (1980)]. In the studies of a rat brain in 1985, there has been elucidated that NGF is a neurotrophic factor at this site of the brain [*EMBO J*., Vol. 4, 1389 (1985)], so that the association of NGF with this disease has been remarked. In addition, there have been elucidated that in the striate body of the brain of a patient with Huntington's chorea, there are remarkable detachment of GABAergic nerve cell as well as detachment of cholinergic nerve cell, so that NGF also acts on the endogenous cholinergic nerve cell of the striate body [*Science*, Vol. 234, 1341 (1986)], addressing a possibility that this disease is associated with NGF. The effects of NGF have been studied with an animal such as a rat which can serve as a model for various nerve diseases. There has been reported that the degeneration of the nerve cell can be stopped in a rat if NGF is intracerebrally administered before the degeneration becomes remarkable, and that the lowering of CAT activity is also prevented [*J. Neurosci*., Vol. 6, 2155 (1986), *Brain Res*., Vol. 293, 305 (1985), *Science,* Vol. 235, 214 (1986), *Proc. Natl. Acad. Sci. USA,* Vol. 83, 9231 (1986)]. Also, it has been proven that NGF is biosynthesized in the peripheral sympathetic nerve-dominant tissues and in the brain, and that each of fibroblasts or astroglia which are interstitial cells for peripheral tissues or brain tissues plays an important role for the NGF biosynthesis [*J*. *Biol*. *Chem*., Vol. 259, 1259 (1984), *Biochem. Biophys. Res. Commun*., Vol. 136, 57 (1986)]. In addition, it has been elucidated that antigenicity, molecular weight, isoelectric point and biological activity of the fibroblast-produced or astroglia-produced NGF are the same as NGF of conventionally well studied submandibular gland. At the same time, it has been found that a catecholamine such as norepinephrine, epinephrine or dopamine shows enhancing effect for NGF production by a test of adding various neurotransmitters to a culture medium of fibroblasts (L-M cells) and astroglia [*J*. *Biol*. *Chem*., Vol. 201, 6039 (1986)].

As described above, there has been expected that NGF can be used as a therapeutic agent for stopping degeneration in a nerve disease in which a site at which NGF acts as a neurotrophic factor is degenerated. In addition, once the cranial nerve cells are degenerated by cebrovascular disorders, cerebral tumor, cerebral apicitus, head injury, nerve degenerative disease, intoxication with an anesthetic, or the like, the degenerated cranial nerve cells would never recover during the life time, whereby various disorders such as emotional disorders and behavioral abnormality are consequently caused in addition to lowering in the intellectual functions and memory disabilities. On the other hand, nerve fiber shows plasticity, that is, when the nerve fiber is damaged, budding takes place from its surrounding healthy fibers, so that a new synapsis is formed in place of the damaged synapsis. Therefore, it has been expected that NGF can be used as a therapeutic agent for promoting restoration and regeneration of nerve functions at this stage.

However, when NGF is applied to a treatment of various nerve diseases, NGF must reach in very close vicinity of nerve cell that requires NGF, and NGF must be also transmitted to lesion site of the brain cell in a case of a disease in the central nervous system. However, NGF cannot be transmitted into the brain through the blood system. This is because the vascular endothelial cells in the brain are bound to each other by adhesion bonding (referred to as brain blood barrier), so that there is a limitation in the transport of a substance other than water, gas or a fat-soluble substance from blood to a brain tissue, whereby a protein (including NGF), which is polymeric substance, cannot pass through the brain blood barrier. There is a too large risk involved in the introduction of NGF directly into the brain by a surgical means, even if the introduction is conducted by the current techniques.

On the other hand, there has been developed a substance for enhancing NGF production, not a direct administration of NGF. Most of the substances, however, have various problems such that the substances have strong toxicity, or the substances have effective concentration very closely approximating concentration at which toxicity is shown, or the substances have severe adverse actions against nervous system such as nerve excitation action. Therefore, these substances have not yet been actually used.

### DISCLOSURE OF INVENTION

An object of the present invention is to provide a medicament, food, beverage or feed, comprising as an effective ingredient an aqueous extract of a mushroom having enhancing action for nerve growth factor production.

As a result of the intensive efforts, the present inventors have found an unexpectedly clearly potent enhancing activity for NGF production in an aqueous extract of a mushroom obtained by using a polar solvent having completely different properties from the nonpolar solvent used in the extraction for the conventional extract derived from a mushroom in the features of the solubility of the substances, especially an aqueous solvent, as compared to that of the conventional extract obtained with the nonpolar solvent.

In other words, summarizing the present invention, a first invention of the present invention relates to a therapeutic agent or prophylactic agent for a disease requiring enhancement of nerve growth factor production for treatment or prevention, characterized in that the therapeutic agent or prophylactic agent comprises as an effective ingredient an aqueous extract of a mushroom.

A second invention of the present invention relates to an enhancing agent for nerve growth factor production, characterized in that the enhancing agent comprises as an effective ingredient an aqueous extract of a mushroom.

A third invention of the present invention relates to a food, beverage or feed for enhancing nerve growth factor production, characterized in that the food, beverage or feed comprises as an effective ingredient an aqueous extract of a mushroom.

In the first to third inventions of the present invention, the mushroom is exemplified by a mushroom belonging to Aphyllophorales or a mushroom belonging to Agaricales, especially preferably at least one member selected from the group consisting of *Hericium erinaceum*, *Grifola frondosa*, *Agaricus bisporus, Lentinula edodes, Pleurotus eryngii*, *Flammulina velutipes* and *Hypsizygus marmoreus* are exemplified.

### BEST MODE FOR CARRYING OUT THE INVENTION

In the present specification, the term "enhancing activity for NGF production" and "enhancing action for NGF production" each refers to a function for enhancing NGF production and enhancement of NGF production, and is not intended to particularly strictly distinguish in its meaning. In addition, the term "enhance" encompasses an embodiment in which the amount of the desired substance is increased after the action as compared to that before the action of the effective ingredient according to the present invention, as well as an embodiment in which the desired substance is produced by the action of the effective ingredient according to the present invention (induce). Also, in the present specification, any of the substances listed as an effective ingredient can be used alone or in admixture of two or more kinds in the present invention.

As the mushrooms usable in the present invention, mushrooms belonging to Basidiomycetes and Ascomycetes can be used. As the mushrooms belonging to Basidiomycetes, mushrooms such as a mushroom belonging to Agaricales, a mushroom belonging to Aphyllophorales, a mushroom belonging to Phallales, a mushroom belonging to Rhizopogonaceae, a mushroom belonging to Lycoperdales, a mushroom belonging to Nidulariales, a mushroom belonging to Sclerodermatales, a mushroom belonging to Auriculariales, a mushroom belonging to Tremellales, a mushroom belonging to Dacryomycetales, a mushroom belonging to Ustilaginales, and a mushroom belonging to Uredinales can be used. More concretely, as the mushroom belonging to Agaricales, *Lyophyllum decastes*, *Hypsizygus marmoreus (Lyophyllum ulmarium)*, *Flammulina velutipes, Lentinula edodes, Pleurotus eryngii*, *Lyophyllum shimeji, Pleurotus ostreatus*, *Agaricus bisporus, Pholiota nameko*, *Agaricus blazei Murrill, Tricholoma matsutake*, and the like can be utilized, as the mushroom belonging to Aphyllophorales, *Hericium erinaceum*, *Grifola frondosa*, *Sarcodon scabrosus*, *Mycoleptodonoides aitchisonii*, *Ganoderma lucidum,* and the like can be utilized, and as the mushroom belonging to Phallales, *Dictyophora indusiata*, and the like can be utilized. Especially preferably, *Hericium erinaceum* belonging to Aphyllophorales Hericiaceae, *Grifola frondosa* belonging to Aphyllophorales Polyporaceae *Microporus, Hypsizygus marmoreus* or *Lyophyllum decastes* belonging to Agaricales Tricholomataceae *Lyophyllum, Flammulina velutipes* belonging to Agaricales Tricholomataceae *Flammulina, Lentinula edodes* belonging to Agaricales Pleurotaceae *Lentinus, Pleurotus eryngii* belonging to Agaricales Pleurotaceae *Pleurotus,* and *Agaricus bisporus* belonging to Agaricales Agaricaceae *Agaricus* can be utilized. In addition, as the mushroom belonging to Ascomycetes, mushrooms such as a mushroom belonging to Pezizales, a mushroom belonging to Helotiales, a mushroom belonging to Tuberaceae, a mushroom belonging to Clavicipitales, and the like can be utilized. More concretely, for instance, *Cordyceps sinensis*, *Morchella esculenta*, *Tuber meranosporum*, and the like can be utilized.

In the present invention, various mushrooms as mentioned above can be utilized. As the mushroom, preferably mushrooms belonging to Basidiomycetes, more preferably a mushroom belonging to Aphyllophorales and/or a mushroom belonging to Agaricales are utilized. Even more preferably, at least one member selected from the group consisting of *Hericium erinaceum* (Aphyllophorales), *Grifola frondosa* (Aphyllophorales), *Agaricus bisporus* (Agaricales), *Lentinula edodes* (Agaricales), *Pleurotus eryngii* (Agaricales), *Flammulina velutipes* (Agaricales) and *Hypsizygus marmoreus* (Agaricales) is utilized.

As the mushroom in the present invention, for instance, naturally obtained fruit body of mushrooms, fruit body obtained by artificial production, mycelium obtained by artificial cultivation, or the like can be utilized.

The extraction method of the aqueous extract of the mushroom (hereinafter referred to as an extract in some cases) usable as an effective ingredient in the present invention can be carried out by the following known method. The term "effective ingredient" as used herein means the effective ingredient itself. Concretely explaining the term, although the "aqueous extract" used as an effective ingredient may be, for instance, used as a mixture with an extraction solvent in actual use, the "aqueous extract" does not mean the mixture, but the aqueous extract itself. Therefore, the aqueous extract used as an effective ingredient means the aqueous extract itself (i.e. dried product of the aqueous extract itself).

For instance, a raw material for extraction can be prepared by picking a raw material fruit body of a mushroom, e.g., a fruit body of *Hericium erinaceum*, in a proper timing, and using the fruit body directly or usually pulverizing the fruit body after drying such as air-drying as desired. Alternatively, in the case where the raw material is mycelium, for instance, a product obtained by drying and powdering a mycelium culture medium by a known method can be used. For instance, a powder obtained by drying mycelium culture of *Hypsizygus marmoreus* by spray-drying can be used as a raw material. In addition, the raw material is aged under various conditions and the aged raw material may be used. In the case wherein the raw material is a commercially available pulverized product of a mushroom, the pulverized product can be used directly as a raw material for extraction. The extraction can be carried out by batch method or continuous method with an aqueous solvent. In the present invention, the aqueous extract refers to a substance extracted from the mushroom obtained through a step of extraction with an aqueous solvent. In addition, the aqueous solvent usable as the extraction solvent in the present invention refers to a solvent that at least comprises water and can function as a polar solvent. The aqueous solvent is exemplified by water, an aqueous solution of a water-soluble salt such as sodium chloride, potassium chloride, magnesium chloride or ammonium carbonate, a buffer such as phosphoric acid-sodium phosphate, acetic acid-sodium acetate, tris-hydrochloric acid or carbonic acid-sodium carbonate, an aqueous solution of an inorganic acid such as hydrochloric acid, carbonic acid, sulfuric acid, nitric acid or phosphoric acid, an aqueous solution of an organic acid such as acetic acid, citric acid, lactic acid, succinic acid, ascorbic acid, fumaric acid or malic acid, and an aqueous solution of a surfactant such as saponin or lecithin, and the like. In addition, a mixed solution of a hydrophilic organic solvent including an alcohol such as ethanol or methanol with water can be used as an aqueous solvent. These can be used alone or in admixture of two or more kinds.

The amount of the extraction solvent may be appropriately determined, and the extraction solvent may be usually used in an amount of preferably from 1 to 100 times the weight of the raw material. The extraction temperature may be also appropriately determined according to its purpose. In the case of a water extraction, usually the extraction temperature is preferably from 4° to 130°C, more preferably from 25° to 100°C. The extraction time may be also determined in consideration of the extraction efficiency. Usually, the raw material, the extraction solvent, and the extraction temperature are preferably set in consideration of the extraction efficiency so that the extraction time is preferably from several minutes to several days, more preferably from 5 minutes to 3 hours. The extraction procedure may be carried out with stirring or allowing the mixture to stand still, and the extraction procedure may be repeated several times as desired. The extract may be subjected to such a treatment as filtration, centrifugation, concentration, ultrafiltration, or molecular sieving as desired. The enhancing activity for NGF production of the extract can be conveniently evaluated by the method described in item (2) of Example 1 set forth below.

The extract can take any of the forms of powders, solids, viscous liquids and liquids by carrying out a known processing treatment to the extract after the extraction. In addition, the extract can be granulated by a known process a powdery form, and the extract can be used in the present invention in the form of a granular solid. The granulation process is not particularly limited, and is exemplified by tumbling granulation, agitation granulation, fluidizing bed granulation, airflow granulation, extruding granulation, compression molding granulation, disintegration granulation, spray granulation, spray-drying granulation or the like. In addition, the powdery extract can be used in the present invention in the form of a liquid prepared by, for instance, dissolving or suspending the powdery extract in a liquid, for instance, water, an alcohol or the like.

In addition, the fraction obtained by fractionating the extract by a known method can be used as the extract of the present invention, as long as the fraction has an enhancing action for NGF production of the present invention. The fractionation means include extraction, separation by precipitation, column chromatography, thin-layer chromatography, and the like. The enhancing substance for NGF production can be also isolated by further proceeding the purification of the resulting fraction using the enhancing activity for NGF production as an index. These extracts can be each used alone or in admixture of two or more kinds. Incidentally, in the present invention, extracts obtained by different extraction methods from the same mushroom can be each used alone or in an admixture of two or more kinds.

The extract obtained in the manner as described above can be used as an effective ingredient for the medicament, food, beverage or feed.

The effective ingredient according to the present invention is not especially found to be toxic as described below. Also, there is no risk of the incidence of adverse actions. Therefore, the enhancement for NGF production can be safely and appropriately carried out. Accordingly, the medicament, food, beverage or feed of the present invention each comprising the effective ingredient is effective for treatment or prevention of a disease requiring enhancement of NGF production for treatment or prevention.

NGF is an endogenous growth factor for maintaining viability and functions of nerve cells, elongating nerve cells in accordance with a concentration gradient of NGF, or the like. Therefore, by enhancing the production of NGF, the treatment or prevention of senile dementia such as Alzheimer's disease, peripheral nerve disorder, cerebrovascular disorder, cerebral tumor, cerebral apicitis, nerve degenerative disease caused by head injury, diseases requiring recovery and regeneration of nerve functions, caused by intoxication with an anesthetic, and the like could be carried out. In addition, it is considered to be useful in the treatment or prevention of amyotrophic lateral sclerosis, drug-induced peripheral nerve disorder, diabetic peripheral nerve disorder, Parkinson's disease, sensory nerve disorder, retinitis pigmentosa, macular dystrophy, and the like.

The disease requiring the enhancement of NGF production in the present invention is not particularly limited, as long as the disease can be treated or prevented by enhancing NGF production with a therapeutic agent, a prophylactic agent or the like, each comprising the above-mentioned effective ingredient. Among them, the therapeutic agent or prophylactic agent, which is the medicament of the present invention, is especially effective against various diseases exemplified above which can be treated or prevented by enhancing NGF production.

The therapeutic agent or prophylactic agent of the present invention for a disease requiring enhancement of NGF production includes those formed into a preparation by combining the above-mentioned effective ingredient according to the present invention with a known pharmaceutical carrier.

The therapeutic agent or prophylactic agent of the present invention is usually manufactured by formulating the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like is optionally added thereto, so that a solid agent such as a tablet, a granule, a powder, a fine powder, and a capsule, or a liquid agent such as a common liquid agent, a suspension agent or an emulsion agent can be formed. In addition, there can be also made into a dry product which can be made liquid by adding an appropriate liquid carrier before use, and an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like. There can be utilized as a carrier, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated. In the case of forming into a tablet or pill, for instance, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for instance, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of a non-orally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, an osmotic regulator, a soothing agent, or the like as desired. It is also possible to produce a solid composition which is dissolved in sterile water or a sterile solvent for injection before use.

The external preparation includes solid, semi-solid or liquid preparations for percutaneous administration or transmucosal (oral or intranasal) administration. The external preparation also includes suppositories and the like. For instance, the external preparation may be prepared as liquid preparations including emulsions, suspensions such as lotions, external tinctures, and liquid agents for transmucosal administration; ointments including oily ointments and hydrophilic ointments; medical adhesives for percutaneous administration or transmucosal administration such as films, tapes and poultices; and the like.

Each of the above-mentioned various preparations can be appropriately produced in accordance with conventional methods by utilizing known pharmaceutical carriers and the like. The content of the effective ingredient in the preparation is not particularly limited, as long as the content is in an amount so that the effective ingredient can be preferably administered within the dose described below in consideration of administration form, administration method and the like of the preparation.

The therapeutic agent and prophylactic agent of the present invention are administered via an administration route appropriate for each of the preparation form. The administration method is not limited to specific one. The agent can be administered internally or externally (or topically) or by injection. The injection can be administered, for instance, intravenously, intramuscularly, subcutaneously, intracutaneously, or the like. As to an external preparation, for instance, a suppository may be administered according to its proper administration method.

The dose of the therapeutic agent or prophylactic agent of the present invention is changeable and properly set depending upon its preparation form, administration method, purpose of use, age, body weight, symptom or the like of a patient to which the therapeutic agent or prophylactic agent is applied, or the like. Generally, the dose of the agent, in terms of the dose of the above-mentioned effective ingredient contained in the preparation, is preferably from 0.1 µg to 200 mg/kg weight for adult per day. As a matter of course, the dose varies depending upon various conditions, so that an amount smaller than the dose mentioned above may be sufficient, or an amount exceeding the dose range may be required. Administration may be carried out once or in several divided portions in a day within the desired dose range. The period of administration may be arbitrarily chosen. Also, the therapeutic agent or prophylactic agent of the present invention can be directly orally administered, or the agent can be added to any foodstuffs to take it on a daily basis.

In addition, the present invention can provide an enhancing agent for NGF production comprising the above-mentioned effective ingredient. The enhancing agent may be the above-mentioned effective ingredient itself, or a composition comprising the above-mentioned effective ingredient. The enhancing agent for NGF production may be prepared by, for instance, formulating the above-mentioned effective ingredient with other ingredients which can be used for the same application as the effective ingredient, and forming into a form of reagent usually used according to the above-mentioned process for preparing the therapeutic agent or prophylactic agent. The content of the above-mentioned effective ingredient in the enhancing agent is not particularly limited, as long as the content is in an amount so that the desired effects of the present invention can be exhibited in consideration of administration method, method of use or the like of the enhancing agent. Also, the amount of the enhancing agent used is not particularly limited, as long as the desired effects of the present invention can be exhibited. Especially in the case where the enhancing agent is administered to a living body, the enhancing agent may be preferably used in an amount so that the effective ingredient can be administered within the dose range of the effective ingredient for the above-mentioned therapeutic agent or prophylactic agent. The enhancing agent for NGF production is useful for enhancement of NGF production in a disease requiring enhancement for NGF production. In addition, the enhancing agent is also useful for screening of drugs for diseases associated with NGF. Furthermore, the enhancing agent is useful for functional studies concerning NGF or physical changes in nerve cells.

In addition, the present invention provides a food, beverage or feed for enhancing NGF production in which the above-mentioned effective ingredient is contained, added and/or diluted. Since the food, beverage or feed of the present invention has enhancing action for NGF production, the food, beverage or feed is very useful in amelioration of symptoms or prevention for a disease requiring enhancement for NGF production.

The process for preparing the food, beverage or feed of the present invention is not particularly limited. For instance, formulation, cooking, processing, and the like can be carried out in accordance with those generally employed for foods, beverages or feeds, and the food, beverage or feed of the present invention can be prepared by the general methods for preparing a food, beverage or feed, as long as the resulting food, beverage or feed may contain the above-mentioned effective ingredient of the present invention, wherein the effective ingredient has enhancing action for NGF production.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham, sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor (*shochu*), vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like, in which each of the foods and beverages comprises the above-mentioned ingredient according to the present invention.

In the food or beverage of the present invention, the above-mentioned effective ingredient is contained, added and/or diluted, and its shape is not particularly limited, as long as the effective ingredient is contained in an amount necessary for exhibiting its enhancing action for NGF production. For instance, the shape includes those which can be taken orally such as tablets, granules and capsules.

The content of the above-mentioned effective ingredient in the food or beverage of the present invention is not particularly limited, and the content can be appropriately selected from the viewpoints of sensory aspect and exhibition of activity. The content of the effective ingredient is, for instance, preferably 0.00001% by weight or more, more preferably from 0.0001 to 100% by weight, still more preferably from 0.0006 to 90% by weight of the food. Also, the content of the effective ingredient is, for instance, preferably 0.00001% by weight or more, more preferably from 0.0001 to 10% by weight, still more preferably from 0.0006 to 6% by weight, of the beverage. Also, it is preferred that the food or beverage of the present invention may be taken such that the effective ingredient contained therein is taken, for instance, in an amount of preferably from 0.001 to 100 mg/kg, preferably from 0.1 to 10 mg/kg per day for adult.

In addition, the present invention provides a feed for an organism having an enhancing action for NGF production, wherein the above-mentioned effective ingredient is contained, added and/or diluted in the feed. In another embodiment, the present invention also provides a method of feeding an organism, characterized by administering the above-mentioned effective ingredient to the organism. In still another embodiment, the present invention provides an organism feeding agent characterized in that the organism feeding agent comprises the above-mentioned effective ingredient.

In these inventions, the organism includes, for instance, culturing or breeding animals, pet animals, and the like. The culturing or breeding animal is exemplified by cattle, experimental animals, poultry, pisces, crustaceae or shellfish. The feed is exemplified by a feed for sustenance of and/or improvement in physical conditions, and the like. The organism feeding agent includes immersion agents, feed additives, beverage additives, and the like.

According to these inventions, the same effects can be expected to be exhibited as those of the above-mentioned therapeutic agent or prophylactic agent of the present invention in the organism exemplified above for applying these, on the basis of the enhancing activity for NGF production of the above-mentioned effective ingredient usable in the present invention. In other words, these inventions have a therapeutic or prophylactic effect for a disease requiring an enhancing action for NGF production in the organism.

The above-mentioned effective ingredient usable in the present invention is usually administered in an amount of preferably from 0.01 to 2000 mg per 1 kg of the subject organism per day. For instance, administration can be made by using the feed of the present invention prepared by adding the effective ingredient and mixing the ingredient with a raw material for an artificially formulated feed for applying to the subject animal, or by using the feed prepared by obtaining an organism feeding agent by mixing the effective ingredient of the present invention with a powder raw material for an artificially formulated feed, and thereafter adding the resulting agent and mixing the agent with other raw materials. The content of the above-mentioned effective ingredient in the feed is not particularly limited. The content can be appropriately set in accordance with its purposes, and an appropriate proportion in the feed is from 0.001 to 15% by weight.

The artificially formulated feed includes feeds using animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; vitamins; minerals; amino acids; antioxidants; and the like as raw materials. In addition, feeds for fish such as fish minced meat are also included.

The process for preparing the feed of the present invention is not particularly limited. In addition, the formulation may be in accordance with those of general feeds, as long as the above-mentioned effective ingredient according to the present invention having enhancing action for NGF production is contained in the feed prepared.

Also, the above-mentioned effective ingredient of the present invention having enhancing action for NGF production can be administered by directly adding the above-mentioned effective ingredient to water, seawater, or the like in a pool, a water tank, a water reservoir, or a feeding range, and immersing a subject organism into the resulting solution. The immersion method is especially effective when the amount of intake of the feed of the subject organism is lowered. The concentration of the effective ingredient according to the present invention having enhancing action for NGF production in water or seawater is not particularly limited, and the effective ingredient may be used in accordance with its purposes. It is appropriate that the concentration is preferably from 0.00001 to 1% by weight.

Also, a beverage comprising the above-mentioned effective ingredient according the present invention having enhancing action for NGF production may be given to a subject organism as a feeding drink. The concentration of the effective ingredient usable in the present invention having enhancing action for NGF production in the beverage is not particularly limited, and the effective ingredient may be used in accordance with its purposes. It is appropriate that the concentration is preferably from 0.0001 to 20% by weight. The organism feeding agent, for instance, an immersion agent, a feed additive, or a beverage additive comprising the above-mentioned effective ingredient of the present invention having enhancing action for NGF production may be prepared by known formulation and preparation method. The content of the effective ingredient in the organism feeding agent is not particularly limited, so long as the desired effects of the present invention can be obtained.

The organism to which the present invention can be applied is not limited. The culturing or breeding animals include cattle such as *Equus, Bos, Porcus, Ovis, Copra*, *Camelus,* and *Lama*; experimental animals such as mice, rats, guinea pigs, and rabbits; poultry such as *Chrysolophus*, ducks, *Meleagris,* and *Struthioniformes;* pisces such as *Pagrus, Oplegnathidae, Paralichthys,* plaice, *Seriola*, young *Seriola*, amberjack, *Thunna*, *Caranx delicatissimus*, *Plecoglossus, Salmo •Oncorhynchus*, *Fugu*, *Anguilla, Misguirus,* and *Parasilurus;* Crustaceae such as *Penaidae*, black tiger shrimp, *Penaeus roentalis*, and *Portulus trituberculatus*; and shellfish such as abalones (*awabi*), turban shells, scallops, and oysters; and the pet animals includes dogs, cats, and the like, so that the feed can be widely applied to animals on land and in water.

By allowing a subject organism to take the feed comprising the above-mentioned effective ingredient usable in the present invention having enhancing action for NGF production, or immersing a subject organism into a solution containing the above-mentioned effective ingredient usable in the present invention having enhancing action for NGF production, the physical conditions of the cattle, experimental animals, poultry, pisces, Caustacea, shellfish, pet animals or the like can be well sustained and ameliorated.

As a still another embodiment of the present invention, there is provided use of the above-mentioned effective ingredient according to the present invention in the preparation of a therapeutic agent or prophylactic agent for a disease requiring enhancement of NGF production, an enhancing agent for NGF production, or a food, beverage or feed for enhancing NGF production. The use embodiments include use embodiments of the above-mentioned effective ingredient in the preparation of the therapeutic agent or prophylactic agent, the enhancing agent for NGF production, or the food, beverage or feed for enhancing NGF production of the present invention mentioned above. For instance, as the use of the above-mentioned effective ingredient in the preparation of a therapeutic agent or prophylactic agent for a disease requiring enhancement of NGF production, or an enhancing agent for NGF production, there are exemplified the use in the preparation of a solid agent such as a tablet, a granule, a powder, a fine powder, and a capsule, a liquid agent such as a common liquid agent, a suspension agent, or an emulsion agent, or a dry product which can be liquefied by adding an appropriate carrier before use.

Further, in a still another embodiment of the present invention, there can be provided a method for enhancing NGF production, comprising administering the above-mentioned effective ingredient according to the present invention to an animal. This method can be carried out by administering the above-mentioned effective ingredient, preferably as the above-mentioned enhancing agent for NGF production, to an animal that is predicted to require or requires enhancement of NGF production, wherein the NGF production is enhanced by the administration. The administration method, dose, or the like of the effective ingredient may be similar to that of the above-mentioned enhancing agent for NGF production. In the method for enhancing NGF production, the therapeutic agent or prophylactic agent, or the food, beverage or feed described below of the present invention can be also used. In addition, the term "animal" includes a mammal such as human, dogs, cats, *Bos, Porcus, Equus,* and the like, among which the method is preferably used for human. The method for enhancing NGF production is useful for, for instance, the enhancement of NGF production in a case of treatment or prevention of a disease requiring enhancement for NGF production. In addition, the method is also useful for screening of drugs for diseases associated with NGF. Furthermore, the method is useful for functional studies concerning NGF or physical changes in nerve cells.

No toxicity is found when the above-mentioned effective ingredient usable in the present invention is administered in an amount effective for the exhibition of its action. For instance, incidences of adverse actions are not found when a water extract of *Hericium erinaceum*, *Grifola frondosa*, *Agaricus bisporus, Lentinula edodes, Pleurotus eryngii*, *Flammulina velutipes* or *Hypsizygus marmoreus* is administered to a mouse in a single dose at 1g/kg, and no cases of death are found.

### EXAMPLES

The present invention will be more concretely described hereinbelow by means of the examples, without by no means limiting the scope of the present invention thereto. Unless specified otherwise, "%" in the examples means "% by weight."

### Example 1

(1) Two grams of a product obtained by pulverizing a dried product of *Hericium erinaceum* (manufactured by Watabe Yakuhin Kabushiki Kaisha) was extracted with 40 ml of each kind of solvents, to obtain each of extracts from *Hericium erinaceum*. As the extraction solvent, each of water (60°C), hexane, chloroform, ethyl acetate and ethanol was used. The extraction was carried out by stirring the mixture for 1 hour. As to the extracts obtained with an extraction solvent other than water, the solvent was removed and replaced with 500 µl of DMSO, to give a 80-fold concentrate of the mixture. The water extract was concentrated to a volume of 5 ml, to give a 8-fold concentrate.
(2) Mouse fibroblast L-M cells (ATCC CCL-1.2) were suspended in M199 medium (manufactured by ICN) containing 0.5% bactopeptone (manufactured by GIBCO) so as to have a concentration of 1.5 × 10⁵ cells/ml. The suspension was put in a 96-well plate in an amount of 0.1 ml each well, and the cells were aseptically cultured. After three days of cultivation, the medium was removed and replaced with M199 medium containing 0.5% bovine serum albumin (manufactured by Sigma). Each of the extracts from *Hericium erinaceum* obtained with various solvents in item (1) of Example 1 was added thereto, and the cells were cultured for 20 hours. After the culture was terminated, the concentration of nerve growth factor in the culture medium was assayed by enzyme immunoassay method (NGF Emax Immuno Assay System: manufactured by Promega). The control is the one with no addition of the sample. The enhancing activity for NGF production is expressed when the NGF concentration in the cell culture medium of the control (amount of NGF produced by cells) is defined as 100%. The amount of each of extracts obtained with various solvents added is shown in Table 1 as a ratio of each solvent extract in the cell culture medium. The experiment was carried out twice, and an average value thereof was taken. As a result, it became evident that only the extract from *Hericium erinaceum* has a potent enhancing activity for NGF production. The results are shown in Tables 1 to 5.

**Table 1**

| Enhancing Activity for NGF Production of Water Extract of *Hericium erinaceum* | |
|---|---|
| Amount (%) | Enhancing Activity for NGF Production (%) |
| 0.63 | 367.7 |
| 1.25 | 798.7 |
| 2.5 | 1030.0 |
| 5 | 1289.0 |
| Here, the amount of NGF produced in the control was 0.099 ng/ml. | |

**Table 2**

| Enhancing Activity for NGF Production of Hexane Extract of *Hericium erinaceum* | |
|---|---|
| Amount (%) | Enhancing Activity for NGF Production (%) |
| 0.13 | 136.6 |
| 0.25 | 116.6 |
| 0.5 | 99.0 |
| 1.0 | 100.7 |
| Here, the amount of NGF produced in the control was 0.106 ng/ml. | |

**Table 3**

| Enhancing Activity for NGF Production of Chloroform Extract of *Hericium erinaceum* | |
|---|---|
| Amount (%) | Enhancing Activity for NGF Production (%) |
| 0.13 | 152.9 |
| 0.25 | 112.9 |
| 0.5 | 101.0 |
| 1.0 | 96.6 |
| Here, the amount of NGF produced in the control was 0.106 ng/ml. | |

**Table 4**

| Enhancing Activity for NGF Production of Ethyl Acetate Extract of *Hericium erinaceum* | |
|---|---|
| Amount (%) | Enhancing Activity for NGF Production (%) |
| 0.06 | 121.2 |
| 0.13 | 93.3 |
| 0.25 | 77.3 |
| 0.5 | 79.5 |
| Here, the amount of NGF produced in the control was 0.097 ng/ml. | |

**Table 5**

| Enhancing Activity for NGF Production of Ethanol Extract of *Hericium erinaceum* | |
|---|---|
| Amount (%) | Enhancing Activity for NGF Production (%) |
| 0.13 | 171.1 |
| 0.25 | 117.1 |
| 0.5 | 145.0 |
| 1.0 | 158.4 |
| Here, the amount of NGF produced in the control was 0.097 ng/ml. | |

### Example 2

(1) A product obtained by pulverizing a lyophilized product of *Agaricus bisporus, Lentinula edodes, Pleurotus eryngii*, *Flammulina velutipes, Hypsizygus marmoreus* or *Grifola frondosa* (any of which are fruit bodies) and a dried product of mycelium of *Hypsizygus marmoreus*, were subjected to water extraction in the same manner as that of Example 1, to give water extracts of various mushrooms.
(2) The enhancing activity for NGF production of the water extracts of various mushrooms obtained in item (1) of Example 2 was assayed in the same manner as that of item (2) of Example 1. As a result, it became evident that the water extracts of *Agaricus bisporus, Lentinula edodes, Pleurotus eryngii*, *Flammulina velutipes*, *Hypsizygus marmoreus* or *Grifola frondosa* (any of which are fruit bodies) and mycelium of *Hypsizygus marmoreus* have enhancing activity for NGF production. The results are shown in Table 6. Here, the amount of NGF production in the controls were 0.160 ng/ml for the tests of *Agaricus bisporus* and *Lentinula edodes,* 0.147 ng/ml for the tests of *Pleurotus eryngii* and *Flammulina velutipes,* 0.134 ng/ml for the tests of *Hypsizygus marmoreus* (fruit body) and *Grifola frondosa*, and 0.300 ng/ml for the test of *Hypsizygus marmoreus* (mycelium).

**Table 6**

| Sample | Amount (%) | Enhancing Activity for NGF Production (%) |
|---|---|---|
| Water Extract of *Agaricus bisporus* | 0.165 | 148.0 |
| | 0.313 | 302.7 |
| | 0.625 | 656.3 |
| Water Extract of *Lentinula edodes* | 0.625 | 110.1 |
| | 1.25 | 198.2 |
| | 2.5 | 337.3 |
| Water Extract of *Pleurotus eryngii* | 2.5 | 307.4 |
| | 5.0 | 702.3 |
| | 10.0 | 1474.6 |
| Water Extract of *Flammulina velutipes* | 1.25 | 184.6 |
| | 2.5 | 369.8 |
| | 5.0 | 713.2 |
| Water Extract of *Hypsizygus marmoreus* | 0.313 | 252.8 |
| | 0.625 | 407.2 |
| | 1.25 | 863.9 |
| | 2.5 | 1526.3 |
| Water Extract of *Grifola frondosa* | 0.313 | 479.6 |
| | 0.625 | 697.4 |
| | 1.25 | 808.1 |
| | 2.5 | 1022.9 |
| Water Extract of Mycelium of | 0.5 | 125.3 |
| *Hypsizygus marmoreus* | 1.0 | 208.9 |

### INDUSTRIAL APPLICABILITY

According to the present invention, there is provided a medicament, food, beverage or feed that is effective for a disease requiring enhancement of nerve growth factor production for treatment or prevention, comprising as an effective ingredient an aqueous extract of mushrooms.

The medicament is useful as a therapeutic agent or prophylactic agent for a disease requiring enhancement of nerve growth factor production such as dementia or nerve disorder. According to the food or beverage, by taking as a foodstuff on a daily basis, the symptoms of a disease requiring enhancement of nerve growth factor production can be ameliorated or the like. In addition, the feed is useful for maintaining homeostasis of a living body by enhancing action for nerve growth factor production.

## Claims

1. A therapeutic agent or prophylactic agent for a disease requiring enhancement of nerve growth factor production for treatment or prevention, **characterized in that** the therapeutic agent or prophylactic agent comprises as an effective ingredient an aqueous extract of a mushroom.

2. The therapeutic agent or prophylactic agent according to claim 1, wherein the mushroom is a mushroom belonging to Aphyllophorales and/or a mushroom belonging to Agaricales.

3. The therapeutic agent or prophylactic agent according to claim 2, wherein the mushroom belonging to Aphyllophorales is *Hericium erinaceum* and/or *Grifola frondosa*, and the mushroom belonging to Agaricales is at least one member selected from the group consisting of *Agaricus bisporus, Lentinula edodes, Pleurotus eryngii*, *Flammulina velutipes* and *Hypsizygus marmoreus*.

4. An enhancing agent for nerve growth factor production, **characterized in that** the enhancing agent comprises as an effective ingredient an aqueous extract of a mushroom.

5. The enhancing agent according to claim 4, wherein the mushroom is a mushroom belonging to Aphyllophorales and/or a mushroom belonging to Agaricales.

6. The enhancing agent according to claim 5, wherein the mushroom belonging to Aphyllophorales is *Hericium erinaceum* and/or *Grifola frondosa*, and the mushroom belonging to Agaricales is at least one member selected from the group consisting of *Agaricus bisporus, Lentinula edodes, Pleurotus eryngii*, *Flammulina velutipes* and *Hypsizygus marmoreus*.

7. A food, beverage or feed for enhancing nerve growth factor production, **characterized in that** the food, beverage or feed comprises as an effective ingredient an aqueous extract of a mushroom.

8. The food, beverage or feed according to claim 7, wherein the mushroom is a mushroom belonging to Aphyllophorales and/or a mushroom belonging to Agaricales.

9. The food, beverage or feed according to claim 8, wherein the mushroom belonging to Aphyllophorales is *Hericium erinaceum* and/or *Grifola frondosa*, and the mushroom belonging to Agaricales is at least one member selected from the group consisting of *Agaricus bisporus, Lentinula edodes, Pleurotus eryngii*, *Flammulina velutipes* and *Hypsizygus marmoreus*.
